# EUROPEAN PATENT SPECIFICATION

(11) **EP 2 928 446 B1**
(45) Date of publication and mention of the grant of the patent: **15.08.2018**
(21) Application number: 13801560.7
(22) Date of filing: 04.12.2013
(51) Int. Cl.: A61K 8/35, A61K 8/36, A61K 8/45, A61Q 3/02, A61K 8/73, A61K 8/37, A61K 8/55, A61K 8/87, A61Q 3/00, A61K 8/34, A61K 8/86

(54) **PHOTOCROSSLINKABLE NAIL MAKEUP COMPOSITION**
PHOTOVERNETZBARE NAGELZUSAMMENSETZUNG
COMPOSITION DE VERNIS A ONGLES PHOTORETICULABLE

(30) Priority: 05.12.2012 FR 1261677; 18.01.2013 US 201361754055 P
(43) Date of publication of application: 14.10.2015
(73) Proprietor: L'Oréal, 75008 Paris (FR)
(72) Inventor: KERGOSIEN, Guillaume, F-92370 Chaville (FR); RIACHI, Carl, F-75013 Paris (FR)
(74) Representative: Lavoix
(86) International application number: PCT/EP2013/075546
(87) International publication number: WO 2014/086869

(56) References cited:
- FR-A1- 2 972 634

## Description

The present invention relates to a photocrosslinkable cosmetic composition, particularly for nail and/or false nail makeup and/or care.

More particularly, the present invention relates to a photocrosslinkable cosmetic finishing composition.

The present invention also relates to a nail and/or false nail makeup and/or care method using said composition.

Nail varnish compositions may be used as a base for the varnish (or *base-coat*), as a nail makeup product, or as a finishing composition (or *top-coat*) to be applied on the nail makeup product, or as a cosmetic nail care product. These compositions may be applied onto natural nails as well as onto false nails.

In the field of nail varnishes, liquid cosmetic compositions are known, which, after application on the nail and under the effect of light radiation, undergo *in situ* polymerization and/or crosslinking reactions, resulting in generally crosslinked polymeric networks. Such photopolymerizable and/or photocrosslinkable compositions are described for example in CA 1 306 954, US 5 456 905, US 7 375 144 and FR 2 823 105.

A drawback of these compositions lies in the difficulty removing same during makeup removal.

Furthermore, the stability over time of these photocrosslinkable compositions, both in terms of chipping resistance and gloss, needs to be enhanced further.

Another drawback of these compositions lies in the toxicity of the unsaturated monomers used. Indeed, these highly reactive low molecular weight molecules diffuse readily in the underlying and adjacent substrates where they react with biological molecules.

The aim of the present invention is also to provide a novel photocrosslinkable composition which is easy to use and remove.

The aim of the present invention is also to provide a novel photocrosslinkable compositions exhibiting an enhanced stability over time, in terms of chipping resistance and gloss.

The aim of the present invention is also to provide a photocrosslinkable composition suitable for solving the toxicity problem in respect of the photocrosslinkable cosmetic compositions according to the prior art.

Another aim of the invention is to obtain photocrosslinkable compositions suitable for providing coats having the following properties: stability over time (with a mild etching or without any etching of the nail or false nail before applying the composition), easy makeup removal, high cosmeticity, outstanding makeup result (homogeneous deposition, easy to apply, comfortable to wear) and/or high gloss.

The present invention relates to a photocrosslinkable cosmetic composition, comprising in a physiologically acceptable medium:
- at least one photocrosslinkable urethane (meth)acrylate compound P1 comprising a polyether chain, said compound having a molar mass greater than or equal to 1000 g/mol,
- at least one film-forming polymer P2,
- at least one photoinitiator, and
- at least one volatile solvent chosen from acetone, ethyl acetate, and propyl acetate, said solvent being preferably present in said composition at a content greater than or equal to 20%, preferentially greater than or equal to 30%, by weight in relation to the total weight of said composition,
wherein the proportion of (meth)acrylate monomer is preferably less than or equal to 10% by weight in relation to the total weight of said composition, and
wherein the weight ratio between P1 and P2 is less than or equal 2.0.

The cosmetic compositions according to the invention comprise a physiologically acceptable medium.

The term "physiologically acceptable medium" refers to a medium that is particularly suitable for the application of a composition of the invention onto keratin matter.

The physiologically acceptable medium is generally suitable for the nature of the support to which the composition should be applied, and also for the way in which the composition is to be packaged.

The term "photocrosslinkable compound" refers to an organic compound suitable for crosslinking under the action of a light ray, resulting in a crosslinked polymer network.

### Urethane (meth)acrylate compound

The composition according to the invention comprises at least one photocrosslinkable compound, referred to as P1, which is a urethane (meth)acrylate compound and comprises a polyether chain.

The term "(meth)acrylate monomer" refers to a compound comprising a single (meth)acrylate function according to the formula H₂C=C(R)-C(O)-O-, where R = H or CH₃.

According to one embodiment, the photocrosslinkable composition according to the invention comprises a reduced proportion of (meth)acrylate monomers, i.e. less than 10% by weight in relation to the total weight of said composition. Preferably, this proportion is less than or equal to 5%, preferentially less than or equal to 1% by weight. Advantageously, the composition according to the invention is completely free from (meth)acrylate monomer.

The term "urethane (meth)acrylate compound" refers to any compound comprising at least one urethane function -O-C(O)-NH-, and at least one (meth)acrylate function according to the formula H₂C=C(R)-C(O)-O-, where R = H or CH₃.

The "urethane" function is also referred to as a "carbamate" function.

According to one embodiment, the photocrosslinkable compound P1 is chosen from the group consisting of urethane poly(meth)acrylate compounds, advantageously in the group consisting of urethane di(meth)acrylate compounds, preferably in the group consisting of urethane dimethacrylate compounds.

According to the present invention, the term "poly(meth)acrylate compound" refers to a (meth)acrylate compound comprising a plurality of (meth)acrylate functions.

In this way, the term "poly(meth)acrylate compound" may refer to a compound comprising at least two methacrylate functions, or at least two acrylate functions, or at least one methacrylate function and at least one acrylate function.

Preferably, P1 is a urethane methacrylate compound.

Advantageously, the mean number of (meth)acrylate functions borne by the photocrosslinkable compounds P1 intended to form, after crosslinking, a crosslinked polymeric network, is greater than 1. Indeed, a polymerizable system consisting of molecules each bearing a single (meth)acrylate function forms, after reacting all of said functions, a linear or branched, and not crosslinked, chain macromolecular system. Only the presence of a certain fraction of molecules bearing at least two (meth)acrylate functions and thus acting as a crosslinking agent is suitable for obtaining a crosslinked polymeric system.

In the implementation of the present invention, the mean number of (meth)acrylate functions per molecule of compound P1 is preferably greater than or equal to 2, advantageously ranging from 2 to 6, preferably from 2 to 4.

Preferentially, P1 is a urethane dimethacrylate compound.

The term "urethane dimethacrylate compound" refers to any compound comprising at least one urethane function -O-C(O)-NH-, and two methacrylate functions according to the formula H₂C=C(CH₃)-C(O)-O-.

### Polyether chain

The term "polyether chain" refers to a C₁-C₁₀₀ divalent hydrocarbon radical, interspersed with at least two oxygen atoms.

Preferably, the polyether chain of the compound P1 has a molar mass less than or equal to 1000 g/mol.

Preferentially, the polyether chain generally comprises less than 50 carbon atoms, preferably less than 45 carbon atoms.

According to one embodiment, the polyether chain of the photocrosslinkable compound P2 is of formula -[CₙH₂ₙO]ₘ-, wherein n is an integer ranging from 1 to 6, preferably equal to 2 or 3, and m is an integer ranging from 2 to 50.

It may thus consist for example of a polyoxymethylene chain according to the formula -[CH₂O]ₘ-, a poly(ethyleneglycol) chain according to the formula -[CH₂CH₂O]ₘ-, a poly(propyleneglycol) chain according to the formula -[CH₂CH(CH₃)O]ₘ- or a polytetramethyleneglycol chain according to the formula -[(CH₂)₄O]ₘ-.

According to one preferred embodiment of this alternative embodiment, the polyether chain is such that n = 2 and m ranges from 2 to 20, preferably from 5 to 20.

According to another embodiment, the polyether chain of the photocrosslinkable compound P1 is of formula -[PhO]ₘ-, wherein Ph refers to a phenylene divalent radical, optionally substituted with one or a plurality of C₁-C₆ alkyls and/or halogen atoms, and m is an integer ranging from 2 to 50.

It may thus consist for example of a polyphenylether chain according to the formula -[C₆H₄O]ₘ- or a poly(p-dimethylphenyl)ether chain according to the formula -[(C₆H₂)(CH₃)₂O]ₘ-.

### Compound P1

The compound P1 is of formula (II): 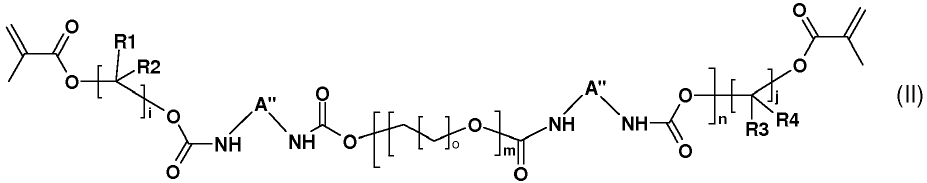 wherein:
- i is an integer ranging from 1 to 6, preferably equal to 2,
- j is an integer ranging from 1 to 6, preferably equal to i, and preferentially equal to 2,
- m is an integer ranging from 1 to 20,
- n is an integer between 1 and 10, preferably equal to 1,
- o is an integer between 1 and 10, preferably equal to 1,
- R1, R3, R3 and R4, identical or different, represent a hydrogen atom or a C₁-C₁₀ alkyl chain, preferably a hydrogen atom or a methyl group.
- -A"- represents a linear or branched C₁-C₂₀ divalent hydrocarbon alkylene group, or a C₅-C₂₀ divalent cycloalkylene radical.

Preferably, -A"- represents a radical according to the formula:

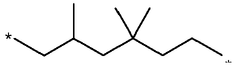

A photocrosslinkable compound suitable for the implementation of the invention is for example PEG 400 Extended Urethane Dimethacrylate (X-726-0000 - ESSTECH, Inc.).

P1 is preferably present at a total content greater than or equal to 1% by weight, in relation to the total weight of the photocrosslinkable composition, advantageously ranging from 1 to 20%, preferably from 2 to 15%, preferably from 5 to 10% by weight in relation to the total weight of the photocrosslinkable composition.

The composition according to the invention optionally comprises a mixture of different compounds P1.

### Film-forming polymer P2

The composition according to the invention comprises at least one film-forming polymer P2, different to the photocrosslinkable compound P1.

Preferably, the film-forming polymer P2 is a non-photocrosslinkable compound.

The term "non-photocrosslinkable compound" refers to a compound inert to light exposure, i.e. that does not polymerize and/or is not crosslinked, unlike the photocrosslinkable compound P1.

In particular, the film-forming polymer P2 is generally free from double ethylene bonds, such as acrylate and methacrylate groups.

The term "film-forming polymer" refers to, according to the invention, a polymer suitable for forming alone (i.e. in the absence of an auxiliary film-forming agent or an external stimulus for example such as UV), a film suitable for being isolated, particularly a continuous adherent film, on a substrate, particularly on nails.

A single film-forming polymer or a mixture of film-forming polymers may be used.

This film-forming polymer may be chosen from the group consisting of radical or polycondensate type synthetic polymers, polymers of natural origin, and mixtures thereof.

A film-forming polymer suitable for the invention may be chosen from polysaccharide derivatives, such as cellulose or guar gum derivatives. One preferential polysaccharide derivative suitable for the invention may be nitrocellulose or a polysaccharide ester or alkylether.

The term "polysaccharide ester or alkylether" refers to a polysaccharide consisting of repeat units comprising at least two identical or different rings and having a degree of substitution per saccharide unit between 1.9 and 3, preferably between 2.2 and 2.9, and more particularly between 2.4 and 2.8. The term substitution refers to the functionalization of hydroxyl groups into ester and/or alkylether functions, and/or the functionalization of carboxyl groups into ester functions.

In other words, it may consist of a polysaccharide, partially or totally substituted with ester and/or alkylether groups. Preferably, the hydroxyl groups may be substituted with C₂-C₄ ester and/or alkylether functions.

Particular mention may be made of cellulose esters (such as cellulose acetobutyrates or cellulose acetopropionates), cellulose alkylethers (such as ethylcelluloses), and ethylguars.

A film-forming polymer suitable for the invention may be chosen from synthetic polymers such as polyurethanes, acrylic polymers, vinyl polymers, polyvinylbutyrals, alkyd resins and ketone/aldehyde resins, resins from aldehyde condensation products, such as aryl sulfonamide formaldehyde resins such as toluene sulfonamide formaldehyde resin, aryl-sulfonamide epoxy resins or ethyl tosylamide resins.

In particular, it may consist of(meth)acrylate homopolymers and copolymers.

A film-forming polymer suitable for the invention may also be chosen from polymers of natural origin, such as plant resins such as dammars, elemi, copals, benzoin; gums such as shellac, sandarac and mastic.

As a film-forming polymer, the toluene sulfonamide formaldehyde resins "Ketjentflex MS80" from AKZO or "Santolite MHP", "Santolite MS 80" from FACONNIER or "RESIMPOL 80" from PAN AMERICANA, the alkyd resin "BECKOSOL ODE 230-70-E" from DAINIPPON, the acrylic resin "ACRYLOID B66" from ROHM & HAAS, the polyurethane resin "TRIXENE PR 4127" from BAXENDEN, the acetophenone/formaldehyde resin marketed under the reference Synthetic Resin SK by Degussa may notably be used.

According to one preferred particular embodiment, the film-forming polymer P2 is chosen from the group consisting of polysaccharides and polysaccharide derivatives, preferably from nitrocellulose and polysaccharide ethers and esters, particularly C₂-C₄, and more preferentially from cellulose acetobutyrates, cellulose acetopropionates, ethylcelluloses, ethylguars, and mixtures thereof.

According to one particularly preferred embodiment, the film-forming polymer P2 is chosen from the group consisting of nitrocellulose, cellulose acetopropionate, cellulose acetobutyrate, and (meth)acrylate homopolymers and copolymers.

Advantageously, the film-forming polymer P2 is nitrocellulose.

P2 is preferably present at a total content greater than or equal to 1% by weight, in relation to the total weight of the photocrosslinkable composition, advantageously ranging from 1 to 30%, preferably from 10 to 30%, preferably from 15 to 25% by weight in relation to the total weight of the photocrosslinkable composition.

The composition according to the invention optionally comprises a mixture of different polymers P2.

According to one embodiment, the composition according to the invention has a weight ratio between P1 and P2 less than or equal to 1.5, preferably less than or equal to 1.0, preferentially ranging from 0.1 to 1.0, and advantageously from 0.2 to 0.5.

### Photoinitiator

The composition according to the invention comprises at least one photoinitiator.

The photoinitiators suitable for use according to the present invention are known in the art and are described, for example in "Les photoinitiateurs dans la reticulation des revêtements", G. Li Bassi, Double Liaison - Chimie des Peintures, No. 361, November 1985, p.34-41; "Applications industrielles de la polymerisation photoinduite", Henri Strub, L'Actualite Chimique, February 2000, p.5-13; and "Photopolymères: considerations théoriques et reaction de prise", Marc, J.M. Abadie, Double Liaison - Chimie des Peintures, No. 435-436, 1992, p.28-34.

These photoinitiators include:
- α-hydroxyketones, marketed for example under the names DAROCUR® 1173 and 4265, IRGACURE® 184, 2959, and 500 by BASF, and ADDITOL® CPK by CYTEC,
- α-aminoketones, marketed for example under the names IRGACURE® 907 and 369 by BASF,
- aromatic ketones marketed for example under the name ESACURE® TZT by LAMBERTI. Mention may also be made of thioxanthones marketed for example under the name ESACURE® ITX by LAMBERTI, and quinones. These aromatic ketones generally require the presence of a hydrogen donor compound such as tertiary amines and particularly alkanolamines. Mention may particularly be made by the tertiary amine ESACURE® EDB marketed by LAMBERTI.
- α-dicarbonyl derivatives of which the most common is benzyl dimethyl ketal marketed under the name IRGACURE® 651 by BASF. Further commercial products are marketed by LAMBERTI under the name ESACURE® KB1, and
- acylphosphine oxides, such as for example bis-acylphosphine oxides (BAPO) marketed for example under the names IRGACURE® 819, 1700, and 1800, DAROCUR® 4265, LUCIRIN® TPO, and LUCIRIN® TPO-L by BASF.

Preferably, the photoinitiator of the composition according to the invention is chosen from the group consisting of α-hydroxyketones, α-aminoketones, aromatic ketones preferably associated with a hydrogen donor compound, aromatic α-diketones, acylphosphine oxides, and mixtures thereof.

A mixture of photoinitiators absorbing light radiation at various wavelengths is preferably used in the photocrosslinkable composition according to the invention. The absorption spectrum of the photocrosslinkable composition can thus be adapted to the emission spectrum of the light sources used.

Preferably, the composition according to the invention comprises a mixture of two different photoinitiators, such as for example a mixture of an α-hydroxyketone and an acylphosphine oxide.

As a photoinitiator mixture, mention may be made of a mixture of IRGACURE® 184 (BASF) and LUCIRIN® TPO-L (BASF).

A particular group of photoinitiators suitable for use in the photocrosslinkable cosmetic compositions according to the present invention is that of copolymerizable photoinitiators. It consists of molecules comprising both a photoinitiator group capable of photoinduced radical splitting and at least one double ethylene bond. The photoinitiators in this group offer the advantage, in relation to the conventional photoinitiators listed above, of being suitable for being incorporated, via the double bond, into the macromolecular system. This possibility reduces the content of free residual photoinitiators not having undergone photoinduced radical splitting and thus enhances the safety of the layer C1.

As examples of such copolymerizable photoinitiators, mention may be made of benzophenone acrylate derivatives marketed by CYTEC under the names EBECRYL® P36, EBECRYL® P37.

In one preferred embodiment of the invention, polymer photoinitiators or photoinitiators bound onto a high molar mass molecule are used. The choice of such a high mass photoinitiator offers the same advantage as selecting only polymeric copolymerizable compounds, i.e. enhanced safety of the photocrosslinkable cosmetic compositions due to the absence of very reactive molecules liable to diffuse to neighboring biological substrates. The mean molar mass by weight of the photoinitiator is preferably at least equal to 500 g/mol.

For example, mention may be made of an α-hydroxyketone oligomer corresponding to the following formula: 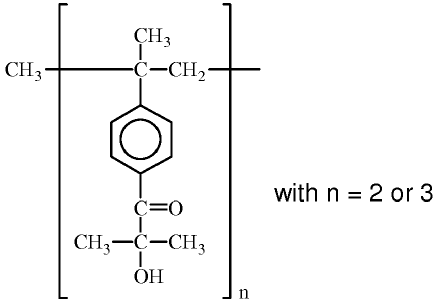 and which is marketed under the name ESACURE® KIP 150 by LAMBERTI.

The polymer on which the photoinitiator group is bound may optionally comprise one or a plurality of double ethylene bonds for optionally incorporating, into the macromolecular network, photoinitiator molecules not having undergone photoinduced splitting.

As examples of such high molar mass photoinitiators bearing double ethylene bonds, mention may be made of those corresponding to the following formulae: 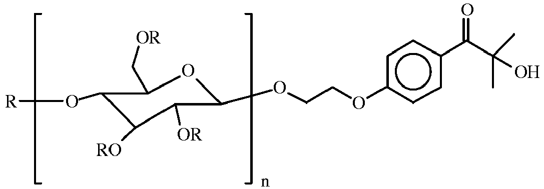 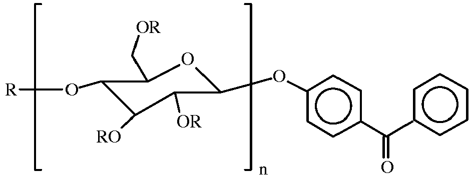 with n = 1 to 20 ; R = H or

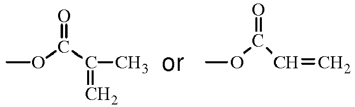

These structures are described in the following articles: S. Knaus, Pure Appl. Chem., A33(7), 869 (1996); S. Knaus, J. Polym. Sci, Part A = Polym. Chem., 33, 929 (1995); and R. Liska, Rad'Tech Europe 97, Lyon, F, 1997, Conference Proceedings.

The photoinitiator content is dependent on a large number of factors such as the reactivity of the various constituents of the mixture, the presence of pigments or dyes, the crosslinking density sought, the intensity of the light source or the exposure time.

In order to obtain satisfactory mechanical properties, the photoinitiator(s) is (or are) preferably present in a total content greater than or equal to 0.1% by weight in relation to the total weight of the photocrosslinkable composition, preferably from 1 to 5% by weight in relation to the total weight of the photocrosslinkable composition.

Preferably, the photoinitiator(s) is (or are) present in a total content greater than or equal to 0.1% by weight in relation to the total weight of the photocrosslinkable compound(s), preferably from 1% to 15% by weight in relation to the total weight of the photocrosslinkable compound(s) P1.

### Solvents

The composition according to the present invention comprises volatile solvents chosen from acetone, ethyl acetate, and propyl acetate. The term "volatile solvent" refers to a solvent capable of evaporating on contact with keratin matter, in less than one hour, at ambient temperature and at atmospheric pressure.

The volatile solvent(s) according to the invention are liquid solvents at ambient temperature, having a vapor pressure different to zero, at ambient temperature and atmospheric pressure, particularly ranging from 0.13 Pa to 40,000 Pa (from 10⁻³ to 300 mm Hg), particularly ranging from 1.3 Pa to 13,000 Pa (from 0.01 to 100 mm Hg), and more specifically ranging from 1.3 Pa to 1300 Pa (from 0.01 to 10 mm Hg).

The total solvent content in the composition may range from 5% to 95% by weight, in relation to the total weight of the composition.

According to one embodiment, the volatile solvent content in the composition ranges from 30% to 90%, preferably from 50% to 80% in relation to the total weight of said composition.

### Adjuvants

The composition according to the invention may further comprise adjuvants, or additives, particularly chosen from pigments and dyes, plasticizers, coalescing agents, preservatives, waxes, thickeners, perfumes, UV filters, cosmetic active substances for nail care, spreading agents, anti-foaming agents and dispersing agents.

The coloring agent(s) is (or are) present in a total content greater than or equal to 0.1% by weight in relation to the total weight of the layer, ranging preferably from 0.1 to 5%, advantageously from 0.2 to 1% by weight in relation to the total weight of the layer.

Obviously, those skilled in the art will take care to choose these optional adjuvants or additives such that the advantageous properties of the composition according to the invention are not, or are practically not, altered by the envisaged addition.

If the composition comprises pigments and/or dyes, it is particularly advisable to adapt the absorption spectrum of the pigments and/or dyes used to that of the photoinitiators, or conversely the absorption spectrum of the photoinitiators to that of the pigments and/or dyes used, so as to prevent both types of compounds from absorbing light at the same wavelengths. Indeed, the absorption of light by the pigments and/or dyes would render the photoinitiators present beyond a specific depth of the coat almost completely ineffective.

Preferably, the composition according to the invention is transparent.

As used herein, the term transparent denotes that the composition has a HAZEBYK index of less than 5 as measured with a KYKHAZEGLOSS type gloss meter.

One particular composition according to the invention comprises, or consists of:
- from 1% to 10%, preferably from 5% to 10%, of a photocrosslinkable urethane dimethacrylate compound comprising a poly(ethyleneglycol) chain,
- from 10% to 30%, preferably from 15% to 25%, of nitrocellulose,
- from 30% to 90%, preferably from 60% to 80%, of ethyl acetate,
- from 0.5% to 5%, preferably from 1% to 3%, of an α-hydroxyketone photoinitiator, and
- from 0.5% to 3%, preferably from 0.5% to 2%, of an acylphosphine oxide photoinitiator.

The composition according to the invention is typically intended to be used as a finishing composition.

In particular, the composition according to the invention is typically intended to be applied onto a nail, or false nail, previously coated with one or a plurality of layers of conventional, optionally colored, nail varnish, or an adhesive flexible material.

As such, the present invention relates to a makeup and/or care method of a nail and/or false nail, comprising the following steps.
a) applying a photocrosslinkable composition according to the invention as defined above onto a nail or false nail coated with a varnish composition or an adhesive flexible material, whereby a coat consisting of at least one layer of said photocrosslinkable composition is deposited, and
b) exposing the coated nail or false nail obtained following step a) to UV or visible light radiation, whereby the photocrosslinking of the photocrosslinkable compound P1 of said composition is carried out.

The radiation suitable for the crosslinking of the photocrosslinkable composition according to the present invention has a wavelength between 210 and 600 nm, preferably between 250 and 420 nm, preferably between 350 and 410 nm. The use of lasers may also be envisaged.

In one preferred embodiment of the invention, a LED lamp or an UV lamp and particularly a mercury vapor lamp, optionally doped with further elements, such as gallium, suitable for modifying the emission spectrum of the light source, is used.

The exposure time of the deposited coat to radiation is dependent on various factors such as the chemical nature and content of the reactive compounds or the crosslinking density sought.

For nail varnishes, it would generally be sought to obtain satisfactory results for an exposure time between 10 seconds and 10 minutes, preferably between 30 seconds and 5 minutes.

Such a method may use a UV lamp having a power of approximately 36 W.

Preferably, the thickness after drying the coat of photocrosslinkable composition deposited in step a) is less than or equal to 100 µm, preferably less than or equal to 50 µm.

The crosslinked coat obtained from the crosslinking in step b) exhibits a significant stability over time, in terms of chipping resistance and gloss, particularly over the course of at least one week. It thus proves to be resistant to water, friction and shocks, and does not exhibit significant wear or chipping in this interval.

This coat is also capable of being solubilized or increasing in volume and thus weight when placed in contact with a standard makeup removal solvent. This ability to be solubilized or swell, displayed by the crosslinked coat, is specifically advantageous for the removal thereof when applied onto the surface of a nail or false nail. Indeed, the coat may be removed readily merely by means of makeup removal using a conventional solvent.

In this way, the composition according to the invention is advantageously suitable for being removed using standard solvents used in the field of nail varnish, and particularly using acetone and ethyl acetate, and mixtures thereof.

The composition according to the invention is easier to remove than existing photocrosslinkable top coats due to a higher proportion of polymer.

The present invention also relates to a makeup removal method of a nail and/or false nail, comprising the application of a makeup removal composition, such as a standard solvent described above, onto a nail or false nail coated with at least one layer obtained by crosslinking a layer of composition according to the invention, whereby said crosslinked layer is removed, optionally along with a layer of varnish situated between the nail or false nail and said crosslinked layer.

The present invention also relates to a kit comprising:
- a photocrosslinkable cosmetic composition according to the invention,
- an abrasive material having a granulometry greater than or equal to 200 µm, preferably less than 300 µm, advantageously comprised from 220 µm to 280 µm, and
- a LED lamp or an UV lamp.

The present invention also relates to a makeup and/or care method of a nail and/or false nail, comprising the following steps:
i) rubbing the surface of a nail or false nail with an abrasive material having a granulometry greater than or equal to 200 µm, preferably less than 300 µm, advantageously comprised from 220 µm to 280 µm,
ii) applying a photocrosslinkable composition according to the invention, whereby a coat consisting of at least one layer of said photocrosslinkable composition is deposited, and
iii) exposing the coated nail or false nail obtained following step ii) to a LED lamp or an UV lamp, whereby the photocrosslinking of the photocrosslinkable composition is carried out.

Usually, the rubbing step is performed for less than 10 seconds, preferably less than 5 seconds, for example for approximately 3 seconds.

Between step i) and step ii), the nail or false nail may be coated with a varnish composition or an adhesive flexible material, so that the photocrosslinkable composition according to the invention is deposited onto the coat of varnish or onto the adhesive flexible material.

### Examples

The present invention will now be illustrated using the following example.

A "Salon effects" adhesive flexible material from Sally Hansen is applied to the nail.

A photocrosslinkable composition according to the invention is then applied onto the nail coated with the adhesive flexible material. After drying for approximately 5 minutes, the nail covered and coated with the photocrosslinkable composition is then crosslinked for 60 seconds under an "OPI GelColor" lamp from OPI (wavelength: 404 nm).

| | |
|---|---|
| PEG 400 Extended Urethane Dimethacrylate (X-726-0000 - ESSTECH, Inc.) | 7% |
| Nitrocellulose with 30% isopropyl alcohol (viscosity: E22 - 1/2s) | 20% |
| Ethyl acetate | 70% |
| Hydroxy Cyclohexylphenyl Ketone photoinitiator (Irgacure 184 - BASF) | 2% |
| Ethyl-2,4,6-Trimethylbenzoylphenylphosphinate photoinitiator (Lucirin TPO-L - BASF) | 1% |

The ingredients of the composition are introduced into an opaque flask and placed under stirring protected from light with a Rayneri laboratory mixer until a homogeneous mixture is obtained. An aluminum foil will have been previously positioned on the top of the container to prevent the solvents from evaporating.

The nail makeup or care material obtained can be removed with a solvent such as acetone.

## Claims

1. Photocrosslinkable cosmetic composition, comprising in a physiologically acceptable medium:
- at least one photocrosslinkable urethane (meth)acrylate compound P1 comprising a polyether chain, said compound having a molar mass greater than or equal to 1000 g/mol,
and wherein P1 is of formula (II): 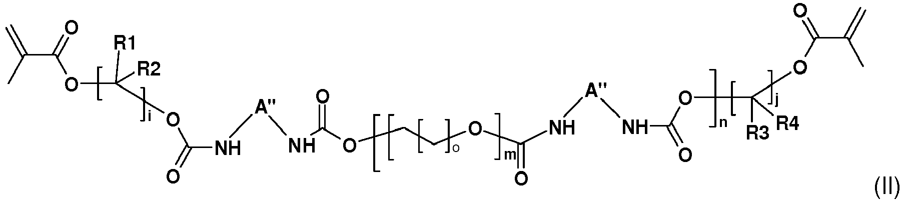 wherein:
- i is an integer ranging from 1 to 6, preferably equal to 2,
- j is an integer ranging from 1 to 6, preferably equal to i, and preferentially equal to 2,
- m is an integer ranging from 1 to 20,
- n is an integer between 1 and 10, preferably equal to 1,
- o is an integer between 1 and 10, preferably equal to 1,
- R1, R3, R3 and R4, identical or different, represent a hydrogen atom or a C₁-C₁₀ alkyl chain, preferably a hydrogen atom or a methyl group, and
- -A"- represents a linear or branched C₁-C₂₀ divalent hydrocarbon alkylene group, or a C₅-C₂₀ divalent cycloalkylene radical.
- at least one film-forming polymer P2,
- at least one photoinitiator, and
- at least one volatile solvent chosen from acetone, ethyl acetate, and propyl acetate, said solvent being preferably present in said composition at a content greater than or equal to 20%, preferentially greater than or equal to 30%, by weight in relation to the total weight of said composition,
wherein the weight ratio between P1 and P2 is less than or equal 2.0.

2. Composition according to claim 1, wherein the polyether chain of P1 has a molar mass less than or equal to 1000 g/mol.

3. Composition according to any of claims 1 or 2, wherein P2 is chosen from the group consisting of nitrocellulose, cellulose acetopropionate, cellulose acetobutyrate, and (meth)acrylate homopolymers and copolymers.

4. Composition according to any of claims 1 to 3, wherein P2 is nitrocellulose.

5. Composition according to any of claims 1 to 4, wherein the weight ratio between P1 and P2 is less than or equal to 1.5.

6. Composition according to any of claims 1 to 5, wherein the photoinitiator is chosen from the group consisting of α-hydroxyketones, α-aminoketones, aromatic ketones preferably associated with a hydrogen donor compound, aromatic α-diketones, and acylphosphine oxides.

7. Composition according to any of claims 1 to 6, wherein the volatile solvent content ranges from 30% to 90% in relation to the total weight of the composition.

8. Composition according to any of claims 1 to 7, **characterized in that** it is transparent.

9. Composition according to any of claims 1 to 8, wherein the proportion of (meth)acrylate monomer is less than or equal to 10% by weight in relation to the total weight of said composition.

10. Makeup and/or care method of a nail and/or false nail, comprising the following steps:
a) applying a photocrosslinkable composition according to the invention as defined in any of claims 1 to 9 onto a nail or false nail coated with a varnish composition or an adhesive flexible material, whereby a coat consisting of at least one layer of said photocrosslinkable composition is deposited, and
b) exposing the coated nail or false nail obtained following step a) to UV or visible light radiation, whereby the photocrosslinking of the photocrosslinkable compound P1 of said composition is carried out.

11. Method according to claim 10, wherein the thickness of the coat of photocrosslinkable composition deposited in step a) is less than or equal to 100 µm.

12. Kit comprising:
- a photocrosslinkable cosmetic composition according to any one of claims 1 to 9,
- an abrasive material having a granulometry greater than or equal to 200 µm, preferably less than 300 µm, advantageously comprised from 220 µm to 280 µm, and
- a LED lamp or an UV lamp.

13. Makeup and/or care method of a nail and/or false nail, comprising the following steps:
i) rubbing the surface of a nail or false nail with an abrasive material having a granulometry greater than or equal to 200 µm, preferably less than 300 µm, advantageously comprised from 220 µm to 280 µm,
ii) applying a photocrosslinkable composition according to any one of claims 1 to 9, whereby a coat consisting of at least one layer of said photocrosslinkable composition is deposited, and
iii) exposing the coated nail or false nail obtained following step ii) to a LED lamp or an UV lamp, whereby the photocrosslinking of the photocrosslinkable composition is carried out.

## Patentansprüche

1. Photovernetzbare kosmetische Zusammensetzung, umfassend, in einem physiologisch verträglichen Medium:
- mindestens eine photovernetzbare Urethan(meth)acrylatverbindung P1 mit einer Polyetherkette, wobei die Verbindung eine Molmasse von größer als oder gleich 1000 g/mol hat,
und wobei P1 der Formel (II) entspricht: 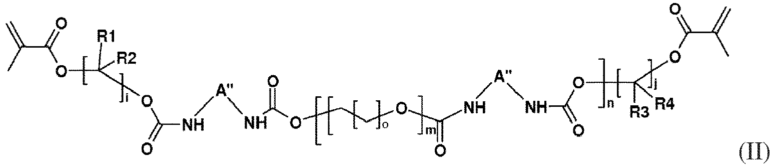 wobei:
- i eine ganze Zahl von 1 bis 6 ist, vorzugsweise gleich 2,
- j eine ganze Zahl von 1 bis 6 ist, vorzugsweise gleich i und bevorzugt gleich 2,
- m eine ganze Zahl von 1 bis 20 ist,
- n eine ganze Zahl zwischen 1 und 10 ist, vorzugsweise gleich 1,
- o eine ganze Zahl zwischen 1 und 10 ist, vorzugsweise gleich 1,
- R1, R3, R3 und R4, gleich oder verschieden, stellen ein Wasserstoffatom oder eine C₁-C₁₀-Alkylkette dar, vorzugsweise ein Wasserstoffatom oder eine Methylgruppe, und
- -A"- stellt eine lineare oder verzweigte C₁-C₂₀ divalente Kohlenwasserstoffalkylengruppe oder einen C₅-C₂₀ divalenten Cycloalkylenrest dar,
- mindestens ein filmbildendes Polymer P2,
- mindestens einen Photoinitiator, und
- mindestens ein flüchtiges Lösungsmittel, ausgewählt aus Aceton, Ethylacetat und Propylacetat, wobei das Lösungsmittel in der Zusammensetzung vorzugsweise in einer Menge von größer als oder gleich 20 Gew.-% vorliegt, bevorzugt größer als oder gleich 30 Gew.-%, bezogen auf das Gesamtgewicht der Zusammensetzung,
wobei das Gewichtsverhältnis zwischen P1 und P2 kleiner als oder gleich 2,0 ist.

2. Zusammensetzung nach Anspruch 1, wobei die Polyetherkette von P1 eine Molmasse von kleiner als oder gleich 1000 g/mol hat.

3. Zusammensetzung nach einem der Ansprüche 1 oder 2, wobei P2 ausgewählt ist aus der Gruppe bestehend aus Nitrocellulose, Celluloseacetopropionat, Celluloseacetobutyrat, und (Meth)Acrylat-Homopolymere und -Copolymere.

4. Zusammensetzung nach einem der Ansprüche 1 bis 3, wobei P2 Nitrocellulose ist.

5. Zusammensetzung nach einem der Ansprüche 1 bis 4, wobei das Gewichtsverhältnis zwischen P1 und P2 kleiner als oder gleich 1,5 ist.

6. Zusammensetzung nach einem der Ansprüche 1 bis 5, wobei der Photoinitiator ausgewählt ist aus der Gruppe bestehend aus α-Hydroxyketonen, α-Aminoketonen, aromatischen Ketonen, vorzugsweise assoziiert mit einer Wasserstoffdonorverbindung, aromatischen α-Diketonen und Acylphosphinoxiden.

7. Zusammensetzung nach einem der Ansprüche 1 bis 6, wobei der Gehalt an flüchtigem Lösungsmittel in einem Bereich von 30% bis 90% liegt, bezogen auf das Gesamtgewicht der Zusammensetzung.

8. Zusammensetzung nach einem der Ansprüche 1 bis 7, **dadurch gekennzeichnet, dass** sie transparent ist.

9. Zusammensetzung nach einem der Ansprüche 1 bis 8, wobei der Anteil an (Meth)acrylatmonomer weniger als oder gleich 10 Gew.-% ist, bezogen auf das Gesamtgewicht der Zusammensetzung.

10. Make-up und/oder Pflegeverfahren eines Nagels und/oder falschen Nagels, umfassend die folgenden Schritte:
a) Aufbringen einer photovernetzbaren Zusammensetzung gemäß der Erfindung, wie in einem der Ansprüche 1 bis 9 definiert, auf einen Nagel oder falschen Nagel, der mit einer Lackzusammensetzung oder einem flexiblen Klebstoff beschichtet ist, wodurch eine Beschichtung, bestehend aus mindestens einer Schicht der photovernetzbaren Zusammensetzung, aufgetragen wird, und
b) Belichten des nach Schritt a) erhaltenen beschichteten Nagels oder falschen Nagels mit UV-Strahlung oder sichtbarem Licht, wodurch die Photovernetzung der photovernetzbaren Verbindung P1 der Zusammensetzung durchgeführt wird.

11. Verfahren nach Anspruch 10, wobei die Dicke der in Schritt a) aufgetragenen Beschichtung aus photovemetzbarer Zusammensetzung kleiner als oder gleich 100 µm ist.

12. Kit umfassend:
- eine photovemetzbare, kosmetische Zusammensetzung nach einem der Ansprüche 1 bis 9,
- ein Schleifmaterial mit einer Granulometrie größer als oder gleich 200 µm, vorzugsweise kleiner als 300 µm, vorteilhafterweise von 220 µm bis 280 µm, und
- eine LED-Lampe oder eine UV-Lampe.

13. Make-up und/oder Pflegeverfahren eines Nagels und/oder falschen Nagels, umfassend die folgenden Schritte:
(i) Reiben der Oberfläche eines Nagels oder falschen Nagels mit einem Schleifmaterial mit einer Granulometrie größer als oder gleich 200 µm, vorzugsweise kleiner als 300 µm, vorteilhafterweise von 220 µm bis 280 µm,
(ii) Aufbringen einer photovernetzbaren Zusammensetzung nach einem der Ansprüche 1 bis 9, wodurch eine Beschichtung, bestehend aus mindestens einer Schicht der photovernetzbaren Zusammensetzung, aufgetragen wird und
(iii) Belichten des nach Schritt ii) erhalten beschichteten Nagels oder falschen Nagels mit einer LED-Lampe oder einer UV-Lampe, wodurch die Photovernetzung der photovernetzbaren Zusammensetzung durchgeführt wird.

## Revendications

1. Composition cosmétique photoréticulable, comprenant dans un milieu physiologiquement acceptable :
- au moins un composé photoréticulable P1 uréthane (méth)acrylate comportant une chaîne polyéther, ledit composé présentant une masse moléculaire supérieure ou égale à 1 000 g/mol, dans laquelle P1 répond à la formule (II) : 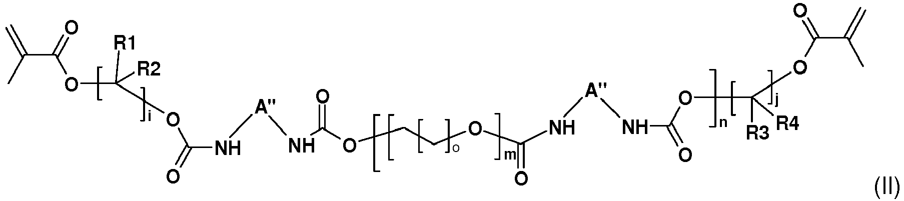 dans laquelle :
- i est un entier compris de 1 à 6, de préférence égal à 2,
- j est un entier compris de 1 à 6, de préférence égal à i, et préférentiellement égal à 2,
- m est un entier compris de 1 à 20,
- n est un entier compris entre 1 et 10, de préférence égal à 1,
- o est un entier compris entre 1 et 10, de préférence égal à 1,
- R1, R2, R3, et R4, identiques ou différents, représentent un atome d'hydrogène ou une chaine alkyle en C₁-C₁₀, de préférence un atome d'hydrogène ou un groupe méthyle.
- -A"- représente un radical divalent hydrocarboné alkylène en C₁-C₂₀, linéaire ou ramifié, ou un radical divalent cycloalkylène en C₅-C₂₀.
- au moins un polymère filmogène P2,
- au moins un photoamorceur, et
- au moins un solvant volatil choisi parmi l'acétone, l'acétate d'éthyle, et l'acétate de propyle, ledit solvant étant de préférence présent dans ladite composition selon une teneur supérieure ou égale à 20%, préférentiellement supérieure ou égale à 30%, en poids par rapport au poids total de ladite composition,
dans laquelle le ratio en poids entre P1 et P2 est inférieur ou égal à 2,0.

2. Composition selon la revendication 1, dans laquelle la chaîne polyéther de P1 présente une masse moléculaire inférieure ou égale à 1 000 g/mol.

3. Composition selon l'une quelconque des revendications 1 ou 2, dans laquelle P2 est choisi dans le groupe constitué de la nitrocellulose, l'acétopropionate de cellulose, l'acétobutyrate de cellulose, et les homopolymères et copolymères (méth)acrylates.

4. Composition selon l'une quelconque des revendications 1 à 3, dans laquelle P2 est la nitrocellulose.

5. Composition selon l'une quelconque des revendications 1 à 4, dans laquelle le ratio en poids entre P1 et P2 est inférieur ou égal à 1,5.

6. Composition selon l'une quelconque des revendications 1 à 5, dans laquelle le photoamorceur est choisi dans le groupe constitué des α-hydroxycétones, des α-aminocétones, des cétones aromatiques de préférence associées à un composé donneur d'hydrogène, des α-dicétones aromatiques, et des oxydes d'acylphosphine.

7. Composition selon l'une quelconque des revendications 1 à 6, dans laquelle la teneur en solvant volatil va de 30% à 90% par rapport au poids total de la composition.

8. Composition selon l'une quelconque des revendications 1 à 7, **caractérisée en ce qu'**elle est transparente.

9. Composition selon l'une quelconque des revendications 1 à 8, dans laquelle la proportion en monomère (méth)acrylate est inférieure ou égale à 10% en poids par rapport au poids total de ladite composition.

10. Procédé de maquillage et/ou de soin des ongles et/ou des faux-ongles, comprenant les étapes suivantes :
a) application d'une composition photoréticulable telle que définie à l'une quelconque des revendications 1 à 9 sur un ongle ou un faux-ongle revêtu d'une composition de vernis à ongles ou d'un article souple adhésif, par laquelle on dépose un revêtement constitué d'au moins une couche de ladite composition photoréticulable, et
b) exposition de l'ongle ou du faux-ongle revêtu obtenu à l'issue de l'étape a) à un rayonnement lumineux, UV ou visible, par laquelle on réalise la photoréticulation du composé photoréticulable P1 de ladite composition.

11. Procédé selon la revendication 10, dans lequel l'épaisseur du revêtement de composition photoréticulable déposé à l'étape a) est inférieure ou égale à 100 µm.

12. Kit comprenant :
- une composition cosmétique photoréticulable selon l'une quelconque des revendications 1 à 9,
- un matériau abrasif présentant une granulométrie supérieure ou égale à 200 µm, de préférence inférieure à 300 µm, avantageusement comprise entre 220 µm et 280 µm, et
- une lampe LED ou une lampe UV.

13. Procédé de maquillage et/ou de soin des ongles et/ou des faux-ongles, comprenant les étapes suivantes :
(i) frotter la surface d'un ongle ou d'un faux-ongle avec un matériau abrasif présentant une granulométrie supérieure ou égale à 200 µm, de préférence inférieure à 300 µm, avantageusement comprise entre 220 µm et 280 µm,
(ii) appliquer une composition photoréticulable selon l'une quelconque des revendications 1 à 9, étape par laquelle un revêtement constitué d'au moins une couche de ladite composition photoréticulable est déposé, et
(iii) exposer l'ongle ou le faux-ongle revêtu, obtenu suite à l'étape (ii), à une lampe LED ou une lampe UV, par laquelle on réalise la photoréticulation de la composition photoréticulable.
